# EUROPEAN PATENT APPLICATION

(11) **EP 2 281 796 A1**
(43) Date of publication of application: **09.02.2011**
(21) Application number: 08874489.1
(22) Date of filing: 26.12.2008
(51) Int. Cl.: C07C 41/03, C07C 43/11, C08G 65/28, C07B 61/00

(54) **METHOD FOR PRODUCING POLYOXYALKYLENE ALKYL ETHER**

(30) Priority: 26.05.2008 JP 2008136421
(71) Applicant: Kao Corporation, Chuo-ku Tokyo 103-8210 (JP)
(72) Inventor: OHTAWA, Yasuki, Wakayama-shi Wakayama 640-8580 (JP); NISHIMOTO, Yoshifumi, Wakayama-shi Wakayama 640-8580 (JP); FUKUSHIMA, Tetsuaki, Wakayama-shi Wakayama 640-8580 (JP)
(74) Representative: HOFFMANN EITLE
(86) International application number: PCT/JP2008/073985
(87) International publication number: WO 2009/144856

(57) **Abstract**

In the present invention, when polyoxyalkylene alkyl ethers are produced by adding propylene oxide to a linear alcohol in the presence of an alkali catalyst, the proportion of the alkali catalyst and the proportion of propylene oxide, per mole of active hydrogen of the linear alcohol, are in specific ranges respectively and the temperature in the addition between the linear alcohol and propylene oxide is in a specific range.

## Description

### Field of the Invention

The present invention relates to a process for producing polyoxyalkylene alkyl ethers.

### Background of the invention

Polyoxyalkylene alkyl ethers are obtained by adding alkylene oxides to an active hydrogen-containing compound in the presence of an alkali metal hydroxide catalyst etc. Generally, alkylene oxides are introduced successively to a reactor charged with an active hydrogen-containing compound as a polymerization initiator with potassium hydroxide as an alkali metal hydroxide catalyst, and then reacted under the conditions of a reaction temperature of 60 to 200°C and the maximum reaction pressure of 0.01 to 1.0 MPa until a predetermined molecular weight is reached, thereby yielding crude polyoxyalkylene alkyl ethers. Then, the catalyst in the crude polyoxyalkylene alkyl ethers may be inactivated either by adsorption filtration with an adsorbent or by neutralization with a mineral acid, an organic acid etc., and may be removed by a post-treatment purification process that involves either dehydration, drying, and filtration of a precipitated potassium salt of the catalyst or water-washing and drying the crude polyoxyalkylene alkyl ethers.

A method of increasing the reaction temperature or the concentration of alkylene oxides during the reaction and a method of increasing the amount of the catalyst are known for increasing the productivity of the polyoxyalkylene alkyl ethers. In these methods, however, it is known that when propylene oxide (also referred to hereinafter as PO) is to be added, an alcoholate formed with an alkali catalyst withdraws a hydrogen atom from a methyl group of PO thereby isomerizing the PO, to form allyl alcohol or propenyl alcohol. The rate of formation of a by-product as alkylene oxide (also referred to hereinafter as AO) isomerization product (also referred to hereinafter as AO isomer) having an allyl or propenyl group at a terminal formed by adding PO to the allyl alcohol or propenyl alcohol is also increased, and thus the amount of the isomerization product is also increased. This AO isomer has a lower molecular weight and broader molecular weight distribution than major polyether polyols and is free of an alkyl chain thus causing performance deterioration. This isomerization product significantly broadens the molecular weight distribution of the whole polyoxyalkylene alkyl ethers and decreases the amount of functional groups (the amount of OH groups). Accordingly, the polyoxyalkylene alkyl ethers containing a large amount of AO isomers undergo significant performance deterioration resulting from a reduction in the number of moles of AO added, and for improving their performance, the number of moles of AO added should increased to a level higher than necessary, but it is unfavorable.

There have been proposed methods of using a double metal cyanide complex (for example, JP-A 3-14812), cesium hydroxide (for example, JP-A 8-134202) or highly pure potassium hydroxide (for example, JP-A 2006-89581) as a polymerization catalyst which is not only able to prevent formation of the AO isomer but also capable of high-speed production of polyoxyalkylene alkyl ether.

GB 1089599 describes a process for producing polyoxyalkylene alkyl ethers, which includes reacting 1,2-propylene oxide with triethylene glycol ethyl ether in a specific molar ratio. JP-A 11-349438 describes a cosmetic emulsifier containing an alkylene oxide derivative.

### Summary of the Invention

The present invention relates to a process for producing polyoxyalkylene alkyl ethers, which includes a step of adding propylene oxide to a linear alcohol in the presence of an alkali catalyst (referred to hereinafter as step (I)), wherein the proportion of the alkali catalyst is more than 0 mol% to 1.5 mol% or less per mole of active hydrogen of the linear alcohol, the proportion of the propylene oxide is 0.1 to 5 moles on the average per mole of active hydrogen of the linear alcohol, and the addition of the propylene oxide to the linear alcohol is conducted at a temperature of 130 to 180°C.

The present invention provides a freezing-point decreasing agent of a linear alcohol, which contains polyoxyalkylene alkyl ethers obtained by the process described above, as well as use of polyoxyalkylene alkyl ethers obtained by the process to decrease the freezing point of a linear alcohol.

### Detailed Description of the Invention

When a double metal cyanide complex is used, there is conventionally a problem that when addition of PO is followed by addition polymerization of ethylene oxide, it is necessary that the double metal cyanide complex be once removed, and then an alkali metal hydroxide, its alkoxide or the like be used again in the polymerization of ethylene oxide.

When a cesium hydroxide catalyst is used, the catalyst is effective only at reaction temperatures in the range of 60 to 98°C in addition-polymerizing propylene oxide, and is not efficient from the viewpoint of productivity. Cesium has a very large molecular weight and thus is low in effect per unit weight, is hardly removable after reaction, and is very highly expensive, so there is also problem from the viewpoint of cost performance.

While the content of sodium hydroxide in potassium hydroxide is usually 1000 to 2000 mg/kg, highly pure potassium hydroxide with 100 mg/kg or less sodium hydroxide is used in JP-A 2006-89581. When such a highly pure potassium hydroxide catalyst is used, there is a problem that the cost of the catalyst is high, and without such special reagent, the intended object cannot be achieved.

In this background, there has been demand for a process wherein the time required for producing polyoxyalkylene alkyl ethers is short and the amount of AO isomers formed during alkylene oxide polymerization is small.

GB 1089599 shows starting materials and manufactured products, both of which are different in structure from the starting materials and the obtained polyoxyalkylene alkyl ethers in the process of the present invention. JP-A 11-349438 shows a catalytic amount different from that of the present invention.

As a result of extensive examination for solving the problems, we found that by using specific reaction conditions, that is, the reaction conditions where the amount of the catalyst used, the amount of propylene oxide used and the reaction temperature are specified respectively, the amount of AO isomers having allyl or propenyl group at the terminal thereof is not increased during a PO addition, even if the reaction temperature is increased to a range in which AO isomers are produced usually with a high possibility.

According to the present invention, the time required for production is short, and the amount of alkylene oxide isomers formed during alkylene oxide polymerization is small.

The process for producing polyoxyalkylene alkyl ethers according to the present invention demonstrates the following effects.
(1) Excellent productivity without necessitating an extra step or an unnecessarily long reaction time.
(2) A small amount of AO isomers and a small amount of particularly the AO isomer having an allyl or propenyl group at the terminal thereof even upon PO addition, and little change in physical properties of reaction products using the polyoxyalkylene alkyl ethers.

The reaction temperature selected in the present invention has been assumed to be in the reaction temperature range where AO isomerization products are obtained generally with high possibility, but the present invention has achieved the unexpected effect that even if such temperature condition is used, the amount of AO isomers is not increased by using the catalyst and PO in amounts in a specific range.

The linear alcohol used in the present invention, although being not particularly limited, is for example an alcohol having a linear alkyl group having 6 to 22 carbon atoms. That is, the linear alcohol is preferably a compound represented by the following general formula (1). The linear alcohol, particularly the linear alkyl group, has 6 to 22 carbon atoms from the viewpoint of use thereof as a surfactant.

R¹-OH (1)

wherein R¹ is a linear alkyl group having 6 to 22 carbon atoms.

One feature of the present invention is that the amount (proportion) of the alkali catalyst used in the step (I) is more than 0 mol% to 1.5 mol% or less per mole of active hydrogen of a linear alcohol. The molar ratio of the catalyst is preferably 1.2 mol% or less, more preferably 1.0 mol% or less, or is preferably 0.01 mol% or more, more preferably 0.05 mol% or more, even more preferably 0.1 mol% or more, and even more preferably 0.2 mol% or more. When the proportion of the alkali catalyst is higher than 1.5 mol% per mole of active hydrogen of a linear alcohol, it becomes difficult to prevent an increase in the amount of AO isomers in the polyoxyalkylene alkyl ethers, while maintaining the degree of polymerization of alkylene oxide.

Although the alkali catalyst used in the process of the present invention is not particularly limited, alkali metal hydroxides such as sodium hydroxide and potassium hydroxide can be used. The catalyst is preferably potassium hydroxide.

Sodium hydroxide and potassium hydroxide may be in any arbitrary state and may be used in a dried state or in the form of an aqueous solution. Sodium hydroxide and potassium hydroxide may be industrially available products, and products of general industrial grade may be used as such.

Another feature of the present invention is that the number of moles of PO added is 0.1 to 5 moles on the average per mole of active hydrogen of a linear alcohol. The molar ratio of PO is preferably 0.1 to 3 moles on the average, more preferably 0.1 to 2 moles on the average, even more preferably 0.1 to 1 mole on the average, per mole of active hydrogen of a linear alcohol. By using PO in this range, the generation of AO isomers can be significantly prevented when the reaction temperature in addition polymerization is increased for increasing productivity.

Another feature of the present invention is that while the amounts of the alkali catalyst and PO used in the step (I) are in specific ranges respectively, addition of propylene oxide to linear alcohol is conducted under the condition of a reaction temperature of 130 to 180°C. The reaction temperature is preferably 130 to 170°C, more preferably 140 to 160°C, even more preferably 150 to 160°C. By conducting the PO addition to linear alcohol at this reaction temperature, the reaction rate is increased and the productivity is improved.

In the step (I), it is preferable that the reaction temperature be 130 to 160°C and the amount of the catalyst be 0.01 or more to 1.5 mol% or less per mole of active hydrogen of a linear alcohol, because the productivity of polyoxyalkylene alkyl ethers is improved while the content of AO isomers is decreased.

In the step (I), it is preferable that the amount of the catalyst is 0.01 or more to 1.5 mol% or less per mole of active hydrogen of a linear alcohol and the proportion of PO be 0.1 to 5 moles on the average per mole of active hydrogen of a linear alcohol, because the content of AO isomers produced along with polyoxyalkylene alkyl ethers is reduced.

In the step (I), it is preferable that the reaction temperature is 130 to 160°C and the proportion of PO is 0.1 to 5 moles on the average per mole of active hydrogen of a linear alcohol, because the productivity of polyoxyalkylene alkyl ethers is improved while the content of AO isomers is decreased.

Particularly in the step (I), it is preferable that the reaction temperature be 130 to 150°C, the amount of the catalyst be 0.01 or more to 1.5 mol% or less per mole of active hydrogen of a linear alcohol, and the proportion of PO be 0.1 to 5 moles on the average per mole of active hydrogen of a linear alcohol, because the productivity of polyoxyalkylene alkyl ethers is improved while the content of AO isomers is decreased.

The reaction pressure between linear alcohol and PO in the step (I), although being not particularly limited, is for example preferably 0.01 to 1.0 MPa, more preferably 0.01 to 0.8 MPa.

In the step (I), it is preferable that the reaction temperature be 130 to 160°C and the reaction pressure be 0.01 to 0.8 MPa, because the productivity of polyoxyalkylene alkyl ethers is improved and the reaction time is reduced.

Particularly in the step (I) in the present invention, it is preferable that the proportion of the alkali catalyst be 0.1 to 1.0 mol% per mole of active hydrogen of the linear alcohol, the proportion of PO be 0.1 to 5 moles on the average per mole of active hydrogen of the linear alcohol, and the addition between the linear alcohol and PO be carried out at a temperature of 130 to 160°C at a pressure of 0.01 to 0.8 MPa, because the productivity of polyoxyalkylene alkyl ethers is improved while the content of AO isomers is reduced.

After the reaction is finished, the catalyst is removed by forming a neutral salt thereof by a known method, for example by adding water and a mineral acid such as hydrochloric acid or phosphoric acid or an organic acid such as acetic acid to the reaction solution, then dehydrating and drying the salt, and removing the precipitated salt of the catalyst through filtration, by a method of absorptive removal by contacting the reaction solution with an absorbent, by a removal method of extracting the catalyst from the reaction solution with either water or water and an organic solvent, or by an ion-exchange method of removing the catalyst with an ion-exchange resin, whereby polyoxyalkylene alkyl ethers are obtained.

The present invention can have step (II) of adding ethylene oxide (referred to hereinafter as EO) to the reaction product obtained in step (I). Polyoxyethylene alkyl ether having PO and EO added in this order to the liner alcohol can thereby be obtained. The step (II) may be conducted immediately after the step (I) or may be conducted for the reaction product after the operations such as neutralization or catalyst removal as described above. Usually, the alkali catalyst used in the step (I) can also be used in the step (II), and thus the method wherein the steps (I) and (II) are successively carried out is advantageous from the viewpoint of efficiency. The amount of EO (number of moles per mole of a linear alcohol) used in the step (II), the reaction temperature and the reaction pressure can be appropriately determined. When the process includes the step (II) , the step (II) can be carried out without particularly adding any additional catalyst as long as the ratio of the alkali catalyst to the liner alcohol is in the predetermined range defined in the step (I) in the present invention. In particular, when the number of moles of EO in the step (II) is 1 to 10 on the average per mole of active hydrogen of the linear alcohol (based on the amount of the linear alcohol used in the step (I)), a sufficient catalytic effect can be attained by the amount of the catalyst used in the step (I).

Generally, a batch reactor is used in industrial production of polyoxyalkylene alkyl ethers, and such batch reactor can also be used in the steps (I) and (II) in the present invention. For example, a high-efficiency agitating tank (Max Blend etc.) equipped with a large special blade, a circulatory reactor etc. can be used.

The steps (I) and (II) can be carried out in the absence of solvent or in the presence of a reaction solvent. When a reaction solvent is used, an active hydrogen-free solvent (acetone, hexane or the like) is preferable.

Hereinafter, the characteristics and physical properties of the polyoxyalkylene alkyl ether obtained by the process of the present invention are described.

For preventing formation of AO isomers, the carbonyl value of the polyoxyalkylene alkyl ether obtained by the process of the present invention is preferably 0.01 to 5 µmol/g, more preferably 0.01 to 2 µmol/g, even more preferably 0.01 to 1 µmol/g.

For preventing formation of AO isomers, the iodine value of the polyoxyalkylene alkyl ether obtained by the process of the present invention is preferably 0.01 to 2.0 g I₂/100 g, more preferably 0.01 to 1.0 g I₂/100 g, even more preferably 0.01 to 0.3 g I₂/100 g, and even more preferably 0.01 to 0.2 g I₂/100 g.

The polyoxyalkylene alkyl ether obtained by the process is not only usable in applications to surfactants, detergents, emulsifiers, solubilizers, dispersants, thickeners, antistatic agents, wetting agents etc., but is also effective as precursors of anionic surfactants, such as polyoxyalkylene alkyl ether sulfonates, polyoxyalkylene alkyl ether sulfonates, and polyoxyalkylene alkyl ether phosphates.

In the present invention, the polyoxyalkylene alkyl ether obtained in the step (I) or (II) in the process of the present invention can be used as a freezing-point decreasing agent of a linear alcohol.

The linear alcohol is a compound represented by, for example, the following general formula (2):

R²-OH (2)

wherein R² is a linear alkyl group having 6 to 22 carbon atoms.

The present invention provides a linear alcohol-containing composition containing 20 to 95 wt% linear alcohol and 5 to 80 wt% freezing-point decreasing agent containing the polyoxyalkylene alkyl ether obtained in the step (I) in the process of the present invention. The linear alcohol/polyoxyalkylene alkyl ether ratio by weight is preferably 3/1 to 1/15. The total amount of the linear alcohol and the polyoxyalkylene alkyl ether is preferably 70 to 100 wt% in the composition.

The present invention also provides a linear alcohol-containing composition containing 5 to 95 wt% linear alcohol and 95 to 5 wt% freezing-point decreasing agent containing the polyoxyalkylene alkyl ether obtained in the step (II) in the process of the present invention. The linear alcohol/polyoxyalkylene alkyl ether ratio by weight is preferably 15/1 to 1/10. The total amount of the linear alcohol and the polyoxyalkylene alkyl ether is preferably 70 to 100 wt% in the composition.

### Examples

Hereinafter, the present invention is described in more detail by the Examples. The Examples are merely illustrative of the present invention and are not intended to limit the present invention.

### Example 1 (Production of the linear alcohol to which PO was added)

A 5-L autoclave equipped with a stirrer, a temperature meter and an automatic introduction device was charged with 1517 g (8.2 mols) of dodecyl alcohol and 9.5 g of 48% aqueous potassium hydroxide solution (0.02 mol (1 mol%) of potassium hydroxide per mol of active hydrogen of dodecyl alcohol) as a catalyst, then the atmosphere in the mixture system was replaced by nitrogen, and the mixture was dehydrated under reduced pressure (1.3 kPa) at 110°C for 0.5 hour. Then, the mixture was reacted while 474 g of PO (1.0 mol on the average per mole of active hydrogen of dodecyl alcohol) was introduced at 155°C at a pressure of 0.1 to 0.4 MPa. After introduction of PO, the mixture was reacted at 155°C. After the PO addition was finished, the unreacted PO was removed under reduced pressure.

4.9 g acetic acid was added to the reaction product for neutralization treatment and then kept it at 80°C for 0.5 hour to give dodecyl alcohol having 1.0 mole of PO added on the average.

For confirming formation of AO isomerization products, the resulting dodecyl alcohol having 1.0 mol PO added on the average was measured for its carbonyl value according to ASTM E411 and for its iodine value according to JIS K 0070. The results are shown in Table 1. "Amount of by-product formed" in the table is a value obtained by converting the iodine value into a value expressed in mol (1 g I₂/100g = 39.4 µmol/g) and then adding this value to the carbonyl value. "Reaction time" in the table is the PO addition time (total of the PO introduction time and the aging time after introduction). In the table, "Catalyst (mol%)" shows the amount (mol%) of the alkali catalyst per mol of active hydrogen of the alcohol, and "Number of moles of PO added" is the number of moles of PO added on the average per mole of active hydrogen of the alcohol (these definitions hereinafter apply).

### Examples 2 to 4, Comparative Examples 1 to 2

The reaction temperature, the amount of the catalyst, and the number of moles of PO added were changed as shown in Table 1, whereby dodecyl alcohol to which PO was added was obtained. Each product was evaluated in the same manner as in Example 1, and the results are shown in Table 1. Since the amount of the catalyst in Comparative Example 1 is large, the amount of AO isomer is large. Since the reaction temperature in Comparative Example 2 is low, the amount of AO isomer is equal to that in Example 1, but the reaction time is longer.

**Table 1**

| | Alcohol | Temper ature (°C) | Catalyst (mol%) | Number of moles of PO added | Reaction temperature (minute) | Carbonyl value (µmol/g) | Iode value (gl₂/100g) | Total of by-products (µmol/g) | Amount of by-products formed per mol of PO (µmol/g) |
|---|---|---|---|---|---|---|---|---|---|
| Example 1 | Dodecyl alcohol | 155 | 1.0 | 1 | 103 | 0.5 | 0.17 | 7.2 | 7.2 |
| Example 2 | Dodecyl alcohol | 155 | 0.5 | 1 | 207 | 0.3 | 0.14 | 5.8 | 5.8 |
| Examples 3 | Dodecyl alcohol | 155 | 0.5 | 0.4 | 89 | 0.3 | 0.01 | 0.7 | 1.7 |
| Examples 4 | Dodecyl alcohol | 145 | 1.0 | 0.4 | 72 | 0.2 | 0.02 | 1.0 | 2.5 |
| Comparative example 1 | Dodecyl alcohol | 155 | 2.0 | 1 | 109 | 0.3 | 0.31 | 12.5 | 12.5 |
| Comparative example 2 | Dodecyl alcohol | 120 | 1.0 | 1 | 299 | 0.1 | 0.20 | 8.0 | 8.0 |

### Examples 5 to 7

The type of alcohol, the reaction temperature, and the number of moles of PO added were changed as shown in Table 2, whereby the alcohol to which PO was added was obtained. The resulting alcohol to which PO had been added was measured for its carbonyl value and iodine value in the same manner as in Example 1. The results are shown in Table 2. The results in Example 1 and Comparative Example 1 are also shown in Table 2.

**Table 2**

| | Alcohol | Temp eratur e (°C) | Catalyst (mol%) | Number of moles of PO added | Reaction time (minut es) | Carbonyl value (µmol/g) | Iodine (gl₂/100) | Total of by-products (µmol/g) | Amount of by-product formed per mol of PO (µmol/g) |
|---|---|---|---|---|---|---|---|---|---|
| Example 1 | Dodecyl alcohol | 155 | 1.0 | 1 | 103 | 0.5 | 0.17 | 7.2 | 7.2 |
| Example 5 | Octyl alcohol | 155 | 1.0 | 2.6 | 200 | 1.4 | 0.58 | 24.2 | 9.3 |
| Example 6 | Dodecyl alcohol | 155 | 1.0 | 5 | 399 | 2.5 | 1.3 | 53.7 | 10.7 |
| Example 7 | Dodecyl alcohol | 145 | 1.0 | 2.6 | 357 | 0.9 | 0.5 | 20.6 | 7.9 |
| Comparative example 1 | Dodecyl alcohol | 155 | 2.0 | 1 | 109 | 0.3 | 0.31 | 12.5 | 12.5 |

### Example 8 (Production of the linear alcohol to which PO and EO were added)

A 11-L autoclave equipped with a stirrer, a temperature meter and an automatic introduction device was charged with 4790 g (24.6 mols) of coconut composition alcohol and 14.5 g of 48% aqueous potassium hydroxide solution (0.005 mol (0.5 mol%) of potassium hydroxide per mol of active hydrogen of coconut composition alcohol) as a catalyst, then the atmosphere in the mixture system was replaced by nitrogen, and the mixture was dehydrated under reduced pressure (1.3 kPa) at 110°C for 0.5 hour. Then, the mixture was reacted while 575 g of PO (0.4 mol on the average per mole of active hydrogen of coconut composition alcohol) was introduced at 155°C at a pressure of 0.1 to 0.4 MPa. After introduction of PO, the mixture was reacted at 155°C. Then, the mixture was reacted while 1625 g of EO (1.5 mols on the average per mole of active hydrogen of coconut composition alcohol) was introduced at 155°C at a pressure of 0.1 to 0.4 MPa. After introduction of EO, the mixture was reacted at 155°C. After the EO addition was finished, the unreacted EO was removed under reduced pressure.

After the reaction was finished, 12.4 g of 90% lactic acid was added to the reaction product for neutralization treatment and then kept it at 80°C for 1 hour to give dodecyl alcohol having 0.4 mole of PO and 1.5 moles of EO added on the average.

The resulting coconut composition alcohol having 0.4 mole of PO and 1. 5 moles of EO added on the average was measured for its carbonyl value and iodine value in the same manner as in Example 1. The results are shown in Table 3. In the table, "Number of moles of EO added" is the number of moles of EO added on the average per mole of active hydrogen of the alcohol.

**Table 3**

| | Alcohol | Catalyst (mol%) | Step (I) (PO addition step) | | | Step (II) (EO addition step) | | | Carbonyl value (µmol/g | Iodine value (gl₂/100g) | Total of by-products (µmol/g) |
|---|---|---|---|---|---|---|---|---|---|---|---|
| | | | Temperature (°C) | Number of moles of PO added | Reaction time (minutes) | Temperature (°C) | Number of moles of EO added | Reaction time (minutes) | | | |
| Example 8 | Coconut composition alcohol | 0.5 | 155 | 0.4 | 78 | 155 | 1.5 | 180 | 0.8 | 0.05 | 2.8 |

## Claims

1. A process for producing polyoxyalkylene alkyl ethers, which comprises a step of adding propylene oxide to a linear alcohol in the presence of an alkali catalyst (referred to hereinafter as step (I)), wherein the proportion of the alkali catalyst is more than 0 mol% to 1.5 mol% or less per mole of active hydrogen of the linear alcohol, the proportion of the propylene oxide is 0.1 to 5 moles on the average per mole of active hydrogen of the linear alcohol, and the addition of the propylene oxide to the linear alcohol is conducted at a temperature of 130 to 180°C.

2. The process according to claim 1, wherein the linear alcohol is a compound represented by the following general formula (1):
R¹-OH (1)
wherein R¹ is a linear alkyl group having 6 to 22 carbon atoms.

3. The process according to claim 1 or 2, which further comprises a step (II) of adding ethylene oxide to the reaction product obtained in the step (I).

4. A freezing-point decreasing agent of a linear alcohol, which comprises polyoxyalkylene alkyl ethers obtained by the process of any one of claims 1 to 3.

5. Use of polyoxyalkylene alkyl ethers obtained by the process of any one of claims 1 to 3 to decrease the freezing point of a linear alcohol.
